# EUROPEAN PATENT SPECIFICATION

(11) **EP 1 896 012 B1**
(45) Date of publication and mention of the grant of the patent: **24.09.2014**
(21) Application number: 06744244.2
(22) Date of filing: 14.06.2006
(51) Int. Cl.: C07D 211/76, C07D 207/26, C07D 225/02, A61P 29/00, A61P 25/28, A61P 37/00, A61P 19/00

(54) **Anti-inflammatory agents**
Entzündungshemmende Mittel
Agents anti-inflammatoires

(30) Priority: 15.06.2005 GB 0512238
(43) Date of publication of application: 12.03.2008
(60) Divisional application: 10011424.8
(73) Proprietor: Cambridge Enterprise Limited, Cambridge CB1 1TN (GB)
(72) Inventor: GRAINGER, David John, Cambridge CB2 2QQ (GB); FOX, David John, Coventry CV4 7AL (GB)
(74) Representative: Roberts, Michael Austin
(86) International application number: PCT/GB2006/002218
(87) International publication number: WO 2006/134385

(56) References cited:
- EP-A1- 0 519 877
- WO-A1-2006/134384
- GB-A- 1 374 365
- US-A1- 2002 025 957
- CLARKE D R ET AL: "Synthesis and mass spectra of some 3-acylamino-2-piperidones" JOURNAL OF THE CHEMICAL SOCIETY C: ORGANIC, 1971, pages 3743-3748, XP009074602 ISSN: 0022-4952
- GAO XURI ET AL: "Catalytic asymmetric synthesis of a potent thiomarinol antibiotic" JOURNAL OF THE AMERICAN CHEMICAL SOCIETY, vol. 127, no. 6, 16 February 2005 (2005-02-16), pages 1628-1629, XP002406117 ISSN: 0002-7863
- BATRAKOV S. G. ET AL: "A new ornithine-containing lipid from Actinomyces no. 660-15" CHEMISTRY OF NATURAL COMPOUNDS, vol. 8, no. 2, March 1974 (1974-03), pages 153-159, XP001248008 ISSN: 0009-3130
- YING J ET AL: "Structural Identification of Cepaciamide A, a Novel Fungitoxic Compound from Pseudomonas cepacia D-202" TETRAHEDRON LETTERS, vol. 37, no. 7, 12 February 1996 (1996-02-12), pages 1039-1042, XP004030222 ISSN: 0040-4039
- SCHREIBER J ET AL: "The Reaction of Cyanogen Bromide with Mono- and Diamino Acids" JOURNAL OF THE AMERICAN CHEMICAL SOCIETY, vol. 86, no. 12, 20 June 1964 (1964-06-20), pages 2441-2445, XP002406099 ISSN: 0002-7863
- FRINGUELLI RENATA ET AL: "Synthesis and evaluation of anti-apoptotic activity of L-carnitine cyclic analogues and amino acid derivatives." IL FARMACO, vol. 59, no. 4, April 2004 (2004-04), pages 271-277, XP002406077 ISSN: 0014-827X
- COTMAN C W ET AL: "A Potential Role for Apoptosis in Neurodegeneration and Alzheimer's Disease" MOLECULAR NEUROBIOLOGY, vol. 10, no. 1, 1995, pages 19-45, XP009074701 ISSN: 0893-7648
- FOX D J ET AL: "Identification of 3-(Acylamino)azepan-2-ones as Stable Broad-Spectrum Chemokine Inhibitors Resistant to Metabolism in Vivo", JOURNAL OF MEDICINAL CHEMISTRY, AMERICAN CHEMICAL SOCIETY, US, vol. 48, 12 January 2005 (2005-01-12), pages 867-874, XP002358738, ISSN: 0022-2623, DOI: 10.1021/JM049365A

## Description

The invention relates to the use of 3-aminolactam compounds for preparing a medicament intended to prevent or treat inflammatory disorders.

Inflammation is an important component of physiological host defence. Increasingly, however, it is clear that temporally or spatially inappropriate inflammatory responses play a part in a wide range of diseases, including those with an obvious leukocyte component (such as autoimmune diseases, asthma or atherosclerosis) but also in diseases that have not traditionally been considered to involve leukocytes (such as osteoporosis or Alzheimer's disease).

The chemokines are a large family of signalling molecules with homology to interleukin-8 which have been implicated in regulating leukocyte trafficking both in physiological and pathological conditions. With more than fifty ligands and twenty receptors involved in chemokine signalling, the system has the requisite information density to address leukocytes through the complex immune regulatory processes from the bone marrow, to the periphery, then back through secondary lymphoid organs. However, this complexity of the chemokine system has at first hindered pharmacological approaches to modulating inflammatory responses through chemokine receptor blockade. It has proved difficult to determine which chemokine receptor(s) should be inhibited to produce therapeutic benefit in a given inflammatory disease.

More recently, a family of agents which block signalling by a wide range of chemokines simultaneously has been described: Reckless et al., Biochem J. (1999) 340:803-811. The first such agent, a peptide termed "Peptide 3", was found to inhibit leukocyte migration induced by 5 different chemokines, while leaving migration in response to other chemoattractants (such as fMLP or TGF-beta) unaltered. This peptide, and its analogs such as NR58-3.14.3 (i.e. Sequence ID No.1 c(DCys-DGln-DIle-DTrp-DLys-DGln-DLys-DPro-DAsp-DLeu-DCys)-NH₂), are collectively termed "Broad Spectrum Chemokine Inhibitors" (BSCIs). Grainger et al., Biochem. Pharm. 65 (2003) 1027-1034 have subsequently shown BSCIs to have potentially useful anti-inflammatory activity in a range of animal models of diseases. Interestingly, simultaneous blockade of multiple chemokines is not apparently associated with acute or chronic toxicity, suggesting this approach may be a useful strategy for developing new anti-inflammatory medications with similar benefits to steroids but with reduced side-effects.

However, peptides and peptoid derivatives such as NR58-3.14.3, may not be optimal for use in vivo. They are quite expensive to synthesise and have relatively unfavourable pharmacokinetic and pharmacodynamic properties. For example, NR58-3.14.3 is not orally bioavailable and is cleared from blood plasma with a half-life period of less than 30 minutes after intravenous injection.

Two parallel strategies have been adopted to identify novel preparations which retain the anti-inflammatory properties of peptide 3 and NR58-3.14.3, but have improved characteristics for use as pharmaceuticals. Firstly, a series of peptide analogs have been developed, some of which have longer plasma half-lives than NR58-3.14.3 and which are considerably cheaper to synthesise. Secondly, a detailed structure: activity analysis of the peptides has been carried out to identify the key pharmacophores and design small non-peptidic structures which retain the beneficial properties of the original peptide.

This second approach yielded several structurally distinct series of compounds which retained the anti-inflammatory properties of the peptides, including 16-amino and 16-aminoalkyl derivatives of the alkaloid yohimbine, as well as a range of N-substituted 3-aminoglutarimides. (Reference: Fox et al., J. Med Chem 45(2002) 360-370: WO 99/12968 and WO 00/42071.) All of these compounds are broad-spectrum chemokine inhibitors which retain selectivity over non-chemokine chemoattractants, and a number of them have been shown to block acute inflammation in vivo.

The most potent and selective of these compounds was (S)-3-(undec-10-enoyl)-aminoglutarimide (NR58,4), which inhibited chemokine-induced migration in vitro with an ED₅₀ of 5nM. However, further studies revealed that the aminoglutarimide ring was susceptible to enzymatic ring opening in serum. Consequently, for some applications (for example, where the inflammation under treatment is chronic, such as in autoimmune diseases) these compounds may not have optimal properties, and a more stable compound with similar anti-inflammatory properties may be superior.

As an approach to identifying such stable anlogs, various derivatives of (S)- 3-(undec-10-enoyl)-aminoglutarimide have been tested for their stability in serum. One such derivative, the 6-deoxo analog (S)-3-(undec-10-enoyl)-tetrahydropyridin-2-one, is completely stable in human serum for at least 7 days at 37°C, but has considerably reduced potency compared with the parental molecule.

One such family of stable, broad spectrum chemokine inhibitors (BSCIs) are the 3-amino caprolactams, with a seven-membered monolactam ring. Fox et al., J Med Chem 48(2005) 867-874 identifies a number of 3-(acylamino)azepan-2-ones as stable BSCIs resistant to metablism in vivo. However, further useful anti-inflammatory compounds may be generated from other 3-aminolactams with different ring size.

US2002/0025957 describes lactam inhibitors of Faxtor Xa stated to be useful as anticoagulants and thus for treating cardiovascular disease associated with thrombosis. Intermediate pyrrolidin and piperidine lactams are used to produce the lactam inhibitors.

According to the present invention there is provided compounds of general formula (I), 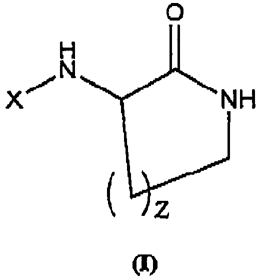 and,
compounds of general formula (I') 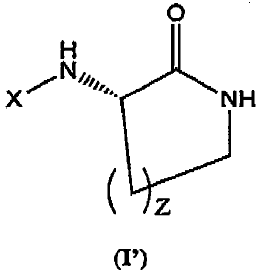 or pharmaceutically acceptable salts of either,
as well as pharmaceutical compositions containing the compounds, and their use for treatment of inflammatory disorders. The scope of the invention (including the terms X and Z in formulae **(I)** and **(I'))** is defined in the appended claims.

The carbon atom at position 3 of the caprolactam ring is asymmetric and consequently, the compounds according to the present invention have two possible enantiomeric forms, that is, the "R" and "S" configurations. The present invention encompasses the two enantiomeric forms and all combinations of these forms, including the racemic "RS" mixtures. With a view to simplicity, when no specific configuration is shown in the structural formulae, it should be understood that the two enantiomeric forms and their mixtures are represented.

By pharmaceutically acceptable salt is meant in particular the addition salts of inorganic acids such as hydrochloride, hydrobromide, hydroiodide, sulphate, phosphate, diphosphate and nitrate or of organic acids such as acetate, maleate, fumarate, tartrate, succinate, citrate, lactate, methanesulphonate, p-toluenesulphonate, palmoate and stearate. Also within the scope of the present invention, when they can be used, are the salts formed from bases such as sodium or potassium hydroxide. For other examples of pharmaceutically acceptable salts, reference can be made to "Salt selection for basic drugs", Int. J. Pharm. (1986), 33, 201-217.

The pharmaceutical composition can be in the form of a solid, for example powders, granules, tablets, gelatin capsules, liposomes or suppositories. Appropriate solid supports can be, for example, calcium phosphate, magnesium stearate, talc, sugars, lactose, dextrin, starch, gelatin, cellulose, methyl cellulose, sodium carboxymethyl cellulose, polyvinylpynolidine and wax. Other appropriate pharmaceutically acceptable excipients and/or carriers will be known to those skilled in the art.

The pharmaceutical compositions according to the invention can also be presented in liquid form, for example, solutions, emulsions, suspensions or syrups. Appropriate liquid supports can be, for example, water, organic solvents such as glycerol or glycols, as well as their mixtures, in varying proportions, in water.

Comparison of several compound series (for example, where X = 2',2'-dimethyldodecanoyl) demonstrates that compounds of formula **(I)** or **(I')** have useful activity irrespective of the size of the lactam ring (z is 1 or 2). For some such compound series the activity of the compounds with z=1 or 2 are essentially indistinguishable. In contrast for other such compound series, the activity of the compound where z=2 is higher than the activity when z=1. Nevertheless, even for such series, the compounds with the least activity still retain sufficient activity to be useful.

In particular, compounds of general formula **(I)** or **(I')** and their salts according to any aspect of the present invention may be selected from the group consisting of:
- (*S*)-3-(2',2'-Dimethyldodecanoylamino)-tetrahydropyridin-2-one;
- (*S*)-3-(2',2'-Dimethyldodecanoylamino)-pyrrolidin-2-one;
and the salts thereof.

Certain alkyl amide derivatives of 3-amino lactams may be known as compounds per se (though it is not presently known that any have been described as such as pharmaceutical compositions or for medical use in an anti-inflammatory context), except in the case of the compound (S)-3-(undec-10-enoylamino)-tetrahydropyridin-2-one which has been described in the literature (Fox et al. J. Med. Chem. (2002) 45:360-70) as broad spectrum chemokine inhibitor in vitro (although it was not tested as to whether it possessed anti-inflammatory activity in vivo), and also undec-10-enoylamino pyrrolidin-2-one (J. Med. Chem. 2005, 48, 867-874).

EP-A1-0519877 discloses a pharmaceutical composition (for enhancing the processes of learning and memory) comprising (3S)-3-(formylamino)-2-pyrrolidinone (ST 879) as the active ingredient.

Fringuelli *et al.* disclose a specific compound laying in the scope of formula I, namely N-(2-oxopiperidin-3-yl)dodecanamide (compound 7, scheme 2), having ability to inhibit Fas-activated apoptosis of human Jurkatt T-cell line and thus showing an anti-apoptotic activity *in in vitro* studies. Furthermore it discloses the implication of apoptosis in the aetiology of several human degenerative neuronal diseases, such as Alzheimer's disease (II Farmaco 2004, 59, 271-277).

The invention includes compounds, compositions and uses thereof as defined, wherein the compound is in hydrated or solvated form.

The amide derivatives of 3-amino lactams described here are functional BSCIs. They are relatively inexpensive to synthesise, using facile synthesis routes provided herein; they are stable in human serum and consequently have excellent pharmacokinetic properties; they are orally bioavailable; they are highly potent broad-spectrum chemokine inhibitors in vitro with excellent selectivity over non-chemokine chemoattractants; they are highly potent and effective anti-inflammatory agents in vivo in rodent models of inflammation; their administration is not associated with any significant acute toxicity at the doses necessary to achieve a maximal therapeutic effect. Taken together, these properties suggest that amide derivatives of 3-amino lactams represent anti-inflammatory medications with advantages over previously described compounds.

In comparison to the prior art the improvement of the present invention lies in the provision of the 3-amino lactam moiety with a side chain having one or more alkyl/alkenyl rings to constrain the bond angles at the alpha carbon of the side chain. Compounds of this invention are significantly superior to compounds with linear alleyl chains (whether alkyl amides or alkyl sulfonamides). In addition, we show that (particularly for compounds with constrained bond angles at the alpha-carbon of the side chain), the size of the lactam ring is relatively unimportant. Variants with five-, six-, seven- and eight-membered lactam rings are all active as BSCIs in vitro, and anti-inflammatory agents in vivo.

Prior art peptides (such as NR58-3.14.3) have the disadvantages that: (a) they are expensive and require solid phase synthesis (at least for the longer ones) and (b) they clear very quickly via the kidneys and (c) they are generally less potent.

The prior art aminoglutarimides are cheap, not cleared quickly via the kidneys and more potent BUT they do not show metabolic stability.

The improvement described here, the aminolactams, are cheap, not cleared by the kidney and even more potent, and are also metabolically stable.

According to this invention, inflammatory disorders intended to be prevented or treated by the compounds of general formula **(I)** or **(I')** or the pharmaceutically acceptable salts thereof or pharmaceutical compositions or medicaments containing them as active ingredients include notably:
- autoimmune diseases, for example such as multiple sclerosis;
- vascular disorders including stroke, coronary artery diseases, myocardial infarction, unstable angina pectoris, atherosclerosis or vasculitis, e. g., Behçet's syndrome, giant cell arteritis, polymyalgia rheumatica, Wegener's granulomatosis, Churg-Strauss syndrome vasculitis, Henoch-Schönlein purpura and Kawasaki disease;
- viral infection or replication, e.g. infections due to or replication of viruses including pox virus, herpes virus (e. g., Herpesvirus samiri), cytomegalovirus (CMV) or lentivirus;
- asthma;
- osteoporosis; (low bone mineral density);
- tumor growth;
- rheumatoid arthritis;
- organ transplant rejection and/or delayed graft or organ function, e.g. in renal transplant patients;
- a disorder characterised by an elevated TNF-α level;
- psoriasis;
- skin wounds;
- disorders caused by intracellular parasites such as malaria or tuberculosis;
- allergies; or
- Alzheimer's disease.

According to this invention, further inflammatory disorders include:
- ALS;
- fibrosis (particularly pulmonary fibrosis, but not limited to fibrosis in the lung);
- the formation of adhesions (particularly in the peritoneum and pelvic region).
- antigen induced recall response
- immune response suppression

These clinical indications fall under the general definition of inflammatory disorders or disorders characterized by elevated TNFα levels.

Administration of a medicament according to the invention can be carried out by topical, oral, parenteral route, by intramuscular injection, etc.

The administration dose envisaged for a medicament according to the invention is comprised between 0.1 mg and 10 g depending on the type of active compound used. According to the invention, the compounds of general formula (I) or **(I')** can be prepared using the processes described hereafter.

### Preparation of the compounds of general formula (I) or (I')

All the compounds of general formula **(I')** or **(I')** can be prepared easily according to general methods known to the person skilled in the art.

Nevertheless, the following preferred synthetic routes are proposed:

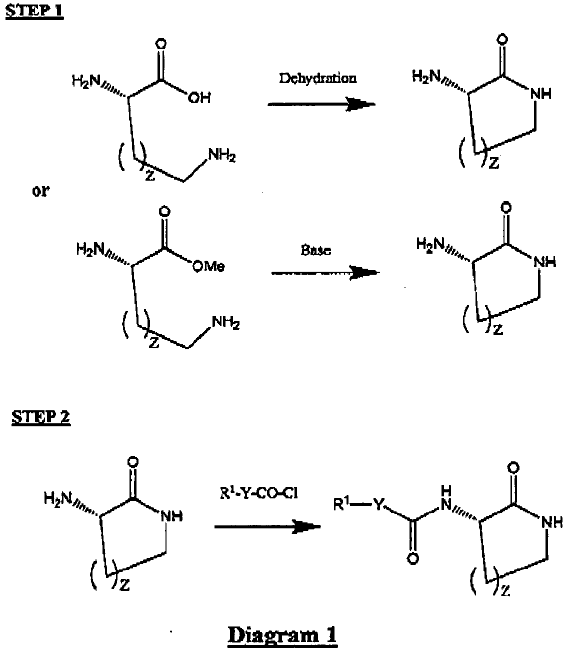

In the first step, 3-aminolactams are synthesised either by direct dehydration of the appropriate diaminocarboxylic acid (2,4-diaminobutyric acid to yield 5-ring amxnolactam, or ornithine to yield a 6-ring lactam) as previously described [Synthesis, 1978, 614-616], or else by base-mediated cyclisation of esters of the same diaminocarboxylic acids, as previously described using lysine methyl ester [J. Org. Chem., 1979, 44, 4841-4847] for the 7-ring lactam.

In the second step, the 3-aminolactam product is reacted with an appropriate acid chloride, for example as previously described for 7-ring aminolactams [J. Med. Chem., 2005, 48, 867-74]. This reaction may be carried out, for example, in chloroform or dichloromethane. The most preferred reaction solvent is dichloromethane, and is preferably carried out in the presence of a base, for example Na₂CO₃.

The above reaction may be carried out at ambient temperature (about 25 °C) or more generally at a temperature between 20 and 50 °C.

The two reactions may be carried out independently, with separation and purification of the 3-aminolactam between the reactions, or alternatively, the reactions may be performed in a single vessel without purification of the 3-aminolactam prior to its derivatisation with acid chloride.

### DEFINITIONS

The term "about" refers to an interval around the considered value. As used in this patent application, "about X" means an interval from X minus 10% of X to X plus 10% of X, and preferably an interval from X minus 5% of X to X plus 5% of X.

The use of a numerical range in this description is intended unambiguously to include within the scope of the invention all individual integers within the range and all the combinations of upper and lower limit numbers within the broadest scope of the given range. Hence, for example, the range of 1 to 20 carbon atoms specified in respect of (*inter alia*) formula I is intended to include all integers between 4 and 20 and all sub-ranges of each combination of upper and lower numbers, whether exemplified explicitly or not.

As used herein, the term "comprising" is to be read as meaning both comprising and consisting of. Consequently, where the invention relates to a "pharmaceutical composition comprising as active ingredient" a compound, this terminology is intended to cover both compositions in which other active ingredients may be present and also compositions which consist only of one active ingredient as defined.

The term "peptidic moieties" used herein is intended to include the following 20 naturally-occurring proteogenic amino acid residues:

| **SYMBOL:** | **MEANING** |
|---|---|
| Ala | Alanine |
| Cys | Cysteine |
| Asp | Aspartic Acid |
| Glu | Glutamic Acid |
| Phe | Phenylalanine |
| Gly | Glycine |
| His | Histidine |
| Ile | Isoleucine |
| Lys | Lysine |
| Leu | Leucine |
| Met | Methionine |
| Asn | Asparagine |
| Pro | Proline |
| Gln | Glutamine |
| Arg | Arginine |
| Ser | Serine |
| Thr | Threonine |
| Val | Valine |
| Trp | Tryptophan |
| Tyr | Tyrosine |

Modified and unusual amino acid residues, as well as peptido-mimetics, are also intended to be encompassed within the definition of "peptidic moieties".

Unless otherwise defined, all the technical and scientific terms used here have the same meaning as that usually understood by an ordinary specialist in the field to which this invention belongs. Similarly, all the publications, patent applications, all the patents and all other references mentioned here are incorporated by way of reference (where legally permissible).

The following examples are presented in order to illustrate the above procedures and should in no way be considered to limit the scope of the invention.

### FIGURES

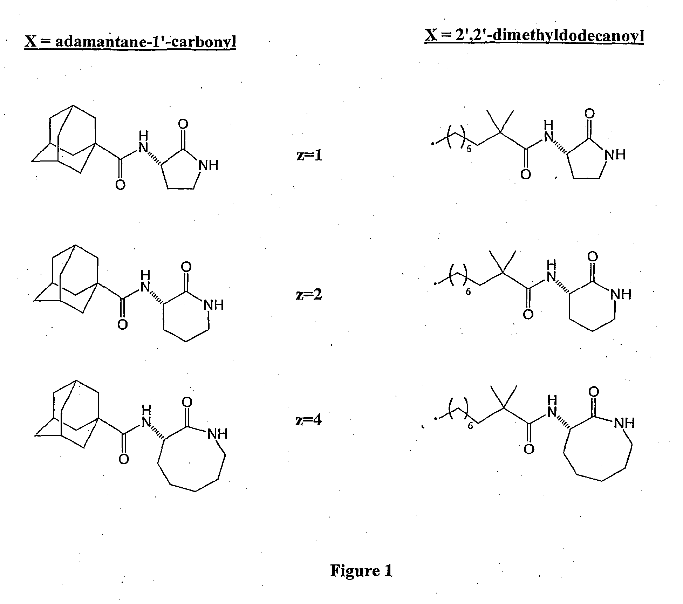

Figure 1 shows the chemical structure of two examples of compounds according to the invention and four reference examples. Two different examples of series of compounds are shown (the series where X = adamantane-1'-carbonyl in the left column and the series where X = 2',2'-dimethyldodecanoyl in the right column). For the series where X = 2',2'-dimethyldodecanoyl, the two possible members claimed here (where z=1 and z=2) are depicted.

### EXAMPLES

3-Aminopyrrolidin-2-one and 3-aminotetrahydropyridin-2-one can be synthesised by direct dehydration of 2,4-diaminobutyric acid and ornithine. [Synthesis, 1978, 614-616] Alternatively the base mediated cyclisation of diamino esters used for the seven-membered lactam [J. Org. Chem., 1979, 44, 4841-4847] can be applied to the synthesis of the five- and six-membered [J. Med. Chem., 2003, 360-370] lactams. Where a single enantiomer of the aminolactam is required, these routes can be performed on enantiomerically pure starting materials, and proceed with retention of stereochemistry.

### Reference Example 1: (S)-3-(1'-Adamantanecarbonylamino)-tetrahydropyridin-2-one:

(*S*)-3-Amino-tetrahydropyridin-2-one hydrochloride (2 mmol) and Na₂CO₃ (6 mmol) in water (25 ml) were added to a solution of adanantane-1-carbonyl chloride (2 mmol) in dichloromethane (25 ml) at ambient temperature and the reaction was stirred for 18 hours. The organic layer was then separated and the aqueous phase was extracted with additional dichloromethane. The combined organic layers were dried over Na₂SO₄ and reduced *in vacuo.* The residue was recrystallised from CH₂Cl₂ / hexanes to give the lactam as an amorphous solid (237 mg. 43%); νₘₐₓ/cm⁻¹ 3330,3175 (NH), 1655,1683 (CO), 1500 (NH); δ_{H} (400 MHz, CDCl₃) 6.59 (1 H, br d, *J* 4.5, NH), 6.51 (1H, br s, NH), 4.15 (1H, dt, *J* 10, 5.5, C*H*NH), 3.35-3.24 (2H, m, C*H*₂NH), 2.57-2,48 (1H, m, lactam CH₂), 1.99 (3H, br s, 3 × adamantane CH), 1.92-1.17 (8H, m, 2 × lactam CH and 6 × adamantane CH₂), 1.73-1.61 (6H, m, 6 × adamantane CH₂) and 1.45 (1H, tt, *J* 12.5, 8.5, lactam CH); δ_{C} (100 MHz, CDCl₃) 178.2, 172.2 (CO), 50.3 (NHCHCO), 41.5 (CH₂N), 40.6 (*C*CO), 39.1 (3 × CH₂ adamantane), 36.5 (3 × CH₂ adamantane), 28.1 (3 × CH adamantane), 27.0, 21.0 (CH₂ lactam); m/z (MNa⁺ C₁₆H₂₄N₂O₂Na requires 299.1735), 299.1739, (MH⁺ C₁₆H₂₅N₂O₂ requires 277.1916) 277.1919.

### Reference Example 2: (S)-3-(1'-Adamantanecarbonylamino)-pyrrolidin-2-one:

(*S*)-3-Amino-pyrrolidin-2-one (2 mmol) and Na₂CO₃ (4 mmol) in water (25 ml) were added to a solution of adamantane-1-carbonyl chloride (2 mmol) in dichloromethane (25 ml) at ambient temperature and the reaction was stirred for 18 hours. The organic layer was then separated and the aqueous phase was extracted with additional dichloromethane. The combined organic layers were dried over Na₂SO₄ and reduced *in vacuo.* The residue was recrystallised from CH₂Cl₂ / hexanes to give the lactam as an amorphous solid (179 mg, 34%); νₘₐₓ/cm⁻¹ 3423, 3233 (NH), 1693, 1664 (CO), 1495 (NH); δ_{H} (400 MHz, CDCl₃) 6.81 (1H, br s, NH), 625 (1H, br s, NH), 4.25 (1H, ddd, *J* 10.5, 8.5, 5, C*H*NH), 3.41-3.28 (2H, m, C*H*₂NH), 2.81-2.71 (1H, m, C*H*₂CH₂N), 2.01 (3H, br s, 3 × adamantane CH), 1.90-1.77 (7H, m, C*H*₂CH₂N and 6 × adamantane CH₂) and 1.73 (3H, br d, *J* 12.5, adamantane CH₂), 1.65 (3H, br d, *J* 12.5, adamantane CH₂); δ_{C} (100 MHz, CDCl₃) 178.7, 176.2 (CO), 50.7 (NHCHCO), 40.6 (CCO), 39.4 (CH₂N), 39.1 (3 × CH₂ adamantane), 36.4 (3 × CH₂ adamantane), 30.3 (CH₂ lactam), 28.1 (3 × CH adamantane); m/z (MNa⁺ C₁₅H₂₂N₂O₂Na requires 285.1579) 285.1577.

### Example 1: (S)-3-(2',2'-Dimethyldadecanoylamino)-tetrahydropyridin-2-one:

(*S*)-3-Amino-tetrahydropyridin-2-one hydrochloride (2 mmol) and Na₂CO₃ (6 mmol) in water (25 ml) were added to a solution of 2,2-dimethyl-dodecanoyl chloride (2 mmol) in dichloromethane (25 ml) at ambient temperature and the reaction was stirred for 18 hours. The organic layer was then separated and the aqueous phase was extracted with additional dichloromethane. The combined organic layers were dried over Na₂SO₄ and reduced *in vacuo.* The residue was purified by silica column chromatography (EtOAc: hexanes 1:3 to MeOH :EtOAc 1:19) to give the lactam as colourless gummy solid (501 mg, 77%); vₘₐₓ/cm⁻¹ 3375 (NH), 1637, 1620 (CO), 1548 (NH); δ_{H} (400 MHz, CDCl₃) 6.62 (1H, br d, J 4.5, NH), 6.34 (1H, br s, NH), 4.17 (1H, dt, *J* 11.5, 5.5, C*H*NH), 3.35-3.24 (2H, m, C*H*₂NH), 2.59-2.5 (1H, m, lactam CH₂), 1.93-1.84 (2H, m, 2 × lactam CH), 1.53-1.40 (3H, m, lactam CH and 2 × sidechain CH₂), 1.30-1.16 (16H, m, (CH₂)₈),1.15 (3H, s, CH₃), 1.14 (3H, s, CH₃) and 0.84 (3H, t, *J* 7, CH₂C*H*₃); δ_{C} (100 MHz, CDCl₃) 178.2, 72.2 (CO), 50.5 (NH*C*HCO), 42.1 (*C*CO), 41.5, 41.4, 31.9, 30.1, 29.6 (x 2), 29.5, 29.3, 27.0 (CH₂), 25.3, 25.2 (CH₃) 24.7, 22.6, 21.0 (CH₂) and 14.1 (CH₃); m/z (MNa⁺ C₁₉H₃₆N₂O₂Na requires 347.2674) 347.2677, (MH⁺ C₁₉H₃₇N₂O₂ requires 325.2855) 325.2863.

### Example 2: (S)-3-(2',2'-Dimethyldodecanoylamino)-pyrrolidin-2-one:

(*S*)-3-Amino-pyrrolidin-2-one (2 mmol) and Na₂CO₃ (4 mmol) in water (25 ml) were added to a solution of 2,2-dimethyl-dodecanoyl chloride (2 mmol) in dichloromethane (25 ml) at ambient temperature and the reaction was stirred for 18 hours. The organic layer was then separated and the aqueous phase was extracted with additional dichloromethane. The combined organic layers were dried over Na₂SO₄ and reduced *in vacuo.* The residue was purified by silica column chromatography (EtOAc: hexanes 1:3 to EtOAc:MeOH 1:19) to give the lactam as a gummy solid (437mg, 70%); νₘₐₓ/cm⁻¹ 3317 (NH), 1704, 1636 (CO), 1531 (NH); δ_{H} (400 MHz, CDCl₃) 6.75 (1H, br s, NH), 6.28 (1H, br d, J4.5, NH), 4.23 (1H, ddd, *J* 10.5, 8.5, 5, C*H*NH), 3.41-3.28 (2H, m, C*H*₂NH), 2.82-2.73 (1H, m, lactam CH₂), 1.85 (1H, dq, *J* 12.5, 9.5, lactam CH₂), 1.50-1.43 (2H, m, 2 × sidechain CH₂), 1.30-1.17 (16H, m, (CH₂)₈), 1.15 (3H, s, CH₃), 1.14 (3H, s, CH₃) and 0.84 (3H, t, *J* 7*,* CH₂C*H*₃); δ_{C} (100 MHz, CDCl₃) 178.7, 176.1 (CO), 50.9 (NHCHCO), 42.0 (*C*CO), 41.3, 39.4, 31.9, 30.2, 30.1, 29.6 (x 2), 29.5, 29.3 (CH₂), 25.3, 25.2 (CH₃) 24.7, 22.6 (CH₂) and 14.1 (CH₃); m/z (MNa⁺ C₁₈H₃₄N₂O₂Na requires 333.2518) 333.2503, (MH⁺ C₁₈H₃₅N₂O₂ requires 311.2699) 311.2693.

### Reference Example 3: (S)-3-(1'-Methylcyclohexanecarbonyl)amino-tetrahydropyridin-2-one

(*S*)-3-Amino-tetrahydropyridin-2-one hydrochloride (2 mmol) and Na₂CO₃ (6 mmol) in water (25 ml) were added to a solution of 1-methylcyclohexanecarbonyl chloride (2 mmol) in dichloromethane (25 ml) at ambient temperature and the reaction was stirred for 18 hours. The organic layer was then separated and the aqueous phase was extracted with additional dichloromethane. The combined organic layers were dried over Na₂SO₄ and reduced *in vacuo.* The residue was purified by silica column chromatography (EtOAc: hexanes 1:3 to MeOH : EtOAc 1:19) to give as an amorphous solid (199 mg, 42%); νₘₐₓ/cm⁻¹ 3335, 3269 (NH), 1650, 1621 (CO), 1529 (NH); δ_{H} (500 MHz, CDCl₃) 6.65 (1H, br d, *J* 5, NH), 6.59 (1H, br s, NH), 4.18 (1H, dt, *J* 11.5, 5.5, C*H*NH), 3.30 (2H, td, *J* 6.5, 2.5, C*H*₂NH), 2.52 (1H, ddt, *J* 13, 5.5, 4.5, lactam CH₂), 1.92-1.83 (4H, m, 2 × lactam CH and 2 × cyclohexane CH₂), 1.55-1.23 (9H, m, lactam CH and 8 × cyclohexane CH₂) and 1.11 (3H, s, CH₃); δ_{C} (125 MHz, CDCl₃) 178.0, 172.3 (CO), 50.4 (NHCHCO), 42.6 (CH₃*C* quat), 41.5, 35.6, 35.5, 27.0 (CH₂), 26.3 (CH₃), 25.7, 22.8 (×2), 20.9 (CH₂); m/z (MNa⁺ C₁₃H₂₂N₂O₂Na requires 261.1579) 261.1570.

### Reference Example 4: (S)-3-(1'-Methylcyclohexanecarbonyl)amino-pyrrolidin-2-one:

(*S*)-3-Amino-pyrrolidin-2-one (2 mmol) and Na₂CO₃ (4 mmol) in water (25 ml) were added to a solution 1-methylcyclohexanecarbonyl chloride (2 mmol) in dichloromethane (25 ml) at ambient temperature and the reaction was stirred for 18 hours. The organic layer was then separated and the aqueous phase was extracted with additional dichloromethane. The combined organic layers were dried over Na₂SO₄ and reduced *in vacuo.* The residue was purified by silica column chromatography (EtOAc: hexanes 1:3 to MeOH : EtOAc 1:19) to give the lactam as an amorphous solid (276 mg, 62%); νₘₐₓ/cm⁻¹ 3321 (NH), 1698, 1633 (CO), 1526 (NH); δ_{H} (400 MHz, CDCl₃) 6.98 (1H, br s, NH), 6.34 (1H, br s, NH), 4.26 (1H, ddd, *J* 10.5, 8.5, 5, C*H*NH), 3.41-3.26 (2H, m, C*H*₂NH), 2.79-2.67 (1H, m, C*H*₂CH₂N), 1.92-1.77 (3H, m, C*H*₂CH₂N and 2 × cyclohexane CH₂), 1.58-1.18 (8H, m, 8 × cyclohexane CH₂) and 1.12 (3H, s, CH₃); δ_{C} (100 MHz, CDCl₃) 178.6, 176.3 (CO), 50.9 (NH*C*HCO), 42.6 (*C*CO), 39.4, 35.5 (×2), 30.0 (CH₂), 26.2 (CH₃), 25.7, 22.8 (x2) (CH₂); m/z (MH⁺ C₁₂H₂₁N₂O₂ requires 225.1603) 225.1596, (MNa⁺ C₁₂H₂₀N₂O₂Na requires 247.1422) 147.1417.

### Reference Example 5: (S)-3-(1'-Phenyleyelohexanecarbonyl)amino-tetrahydropyridin-2-one:

(*S*)-3-Amino-tetrahydropyridin-2-one hydrochloride (2 mmol) and Na₂CO₃ (6 mmol) in water (25 ml) were added to a solution of 1-phenylcyclohexanecarbonyl chloride (2 mmol) in dichloromethane (25 ml) at ambient temperature and the reaction was stirred for 12 hours. The organic layer was then separated and the aqueous phase was extracted with additional dichloromethane (2 × 25 ml). The combined organic layers were dried over Na₂SO₄ and reduced *in vacuo.* The residue was purified crystallisation from hexanes to give the lactam as a solid (327 mg, 54%); νₘₐₓ/cm⁻¹ 3283, 3196 (NH), 1663, 1650 (CO), 1516 (NH); δ_{H} (500 MHz, CDCl₃) 7.43-7.35 (2H, m, Ph), 7.35-7.26 (2H, m, Ph), 7.24-7.17 (1H, m, Ph), 6.48-5.73 (2H, br m, NH), 4.09 (1H, dt, *J* 11, 5.5, C*H*NH), 3.30-3.17 (2H, m, C*H*₂NH), 2.52-2.37 (1H, m, lactam CH), 2.33-2.21 (2H, m, cyclohexane CH), 2.05-1.76 (4H, m, lactam ring CH and cyclohexane CH), 1.65-1.48 (5H, m, cyclohexane CH), and 1.43-1.27 (2H, m, lactam ring CH and cyclohexane CH); δ_{C} (125 MHz, CDCl₃) 175.8, 71.8 (CO), 143.8 (*ipso*-Ph), 128.6 (*ortho-* or *meta*-Ph), 126.6 (*para*-Ph), 126.4 (*ortho*- or *meta*-Ph), 50.8 (NHCHCO), 50.5 (C quat), 41.4 (NCH₂), 34.8, 34.4, 26.7, 25.8, 23.0 (x2), 21.0 (CH₂); m/z (MH⁺ C₁₈H₂₅N₂O₂ requires 301.1916) 301.1905, (MNa⁺ C₁₈H₂₄N₂O₂Na requires 323.1735) 323.1725.

### Pharmacological study of the products of the invention

### Inhibition of MCP-1 induced leukocytes migration

### Assay principle

The biological activity of the compounds of the current invention may be demonstrated using any of a broad range of functional assays of leukocyte migration in vitro, including but not limited to Boyden chamber and related transwell migration assays, under-agarose migration assays and direct visualisation chambers such as the Dunn Chamber.

For example, to demonstrate the inhibition of leukocyte migration in response to chemokines (but not other chemoattractants) the 96-well format micro transwell assay system from Neuroprobe (Gaithersburg, MD, USA) has been used. In principle, this assay consists of two chambers separated by a porous membrane. The chemoattractant is placed in the lower compartment and the cells are placed in the upper compartment.

After incubation for a period at 37°C the cells move towards the chemoattractant, and the number of cells in the lower compartment is proportional to the chemoattractant activity (relative to a series of controls).

This assay can be used with a range of different leukocyte populations. For example, freshly prepared human peripheral blood leukocytes may used. Alternatively, leukocyte subsets may be prepared, including polymorphonuclear cells or lymphocytes or monocytes using methods well known to those skilled in the art such as density gradient centrifugation or magnetic bead separations. Alternatively, immortal cell lines which have been extensively validated as models of human peripheral blood leukocytes may be used, including, but not limited to THP-1 cells as a model of monocytes or Jurkat cells as model of naïve T cells.

Although a range of conditions for the assay are acceptible to demonstrate the inhibition of chemokine-induced leukocyte migration, a specific example is hereby provided.

### Materials

The transwell migration systems are manufactured by Neuroprobe, Gaithersburg, MD, USA.

The plates used are ChemoTx plates (Neuroprobe 101-8) and 30 µl clear plates (Neuroprobe MP30).

Geys' Balanced Salt Solution is purchased from Sigma (Sigma G-9779).

Fatty acid-free BSA is purchased from Sigma (Sigma A-8806).

MTT, i.e. 3-(4,5-dimethylthiazol-2-yl)-2,5-diphenyltetrazolium bromide, is purchased from Sigma (Sigma M-5655).

RPMI-1640 without phenol red is purchased from Sigma (Sigma R-8755).

The THP-1 cell line (European Cell culture Collection) were used as the leukocyte cell population.

### Test protocol

The following procedure is used for testing the invention compounds for MCP-1 induced leukocyte migration:

First, the cell suspension to be placed in the upper compartment is prepared. The THP-1 cells are pelleted by centrifugation (770 x g; 4 mins) and washed with Geys Balanced Salt Solution with 1mg/ml BSA (GBSS + BSA). This wash is then repeated, and the cells repelleted before being resuspended in a small volume of GBSS + BSA for counting, for example using a standard haemocytometer.

The volume of GBSS + BSA is then adjusted depending on the number of cells present so that the cells are at final density of 4.45 x 10⁶ cells per ml of GBSS + BSA. This ensures that there are 100,000 THP-1 cells in each 25 µl of the solution that will be placed in the upper chamber of the plate.

To test a single compound for its ability to inhibit MCP-1 induced migration, it is necessary to prepare two lots of cells. The suspension of THP-1 cells at 4.45 x 10⁶ cells/ml is divided into two pots. To one pot the inhibitor under test is added at an appropriate final concentration, in an appropriate vehicle (for example at 1µM in not more than 1% DMSO). To the second pot an equal volume of GBSS + BSA plus vehicle as appropriate (e.g. not more than 1% DMSO) is added to act as a control.

Next, the chemoattractant solution to be placed in the lower compartment is prepared. MCP-1 is diluted in GBSS + BSA to give a final concentration of 25 ng/ml. This is divided into two pots, as for the cell suspension. To one pot, the test compound is added to the same final concentration as was added to the cell suspension, while to the other pot an equal volume of GBSS + BSA plus vehicle as appropriate (e,g. not more than 1% DMSO) is added.

Note that the volume of liquid that needs to be added to make the addition of the text compound needs to be taken into account, when establishing the final concentration of MCP-1 in the solution for the lower compartment and the final concentration of cells in the upper compartment.

Once the chemoattractant solutions for the lower wells and cell solutios for the upper chambers have been prepared, the migration chamber should be assembled. Place 29 µl of the appropriate chemoattractant solution into the lower well of the chamber. Assays should be performed with at least triplicate determinations of each condition. Once all the lower chambers have been filled, apply the prous membrane to the chamber in accordance with the manufacturer's instructions. Finally, apply 25 µl of the appropriate cell solution to each upper chamber. A plastic lid is placed over the entire apparatus to prevent evaporation.

The assembled chamber is incubated at 37 °C, 5% CO₂, for 2 hours. A suspension of cells in GBSS + BSA is also incubated under identical conditions in a tube: these cells will be used to construct a standard curve for determining the number of cells that have migrated to the lower chamber under each condition.

At the end of the incubation, the liquid cell suspension is gently removed from the upper chamber, and 20µl of ice-cold 20mM EDTA in PBS is added to the upper chamber, and the apparatus is incubated at 4°C for 15 mins. This procedure causes any cells adhering to the underside of the membrane to fall into the lower chamber.

After this incubation the filter is carefully flushed with GBSS + BSA to wash off the EDTA, and then the filter is removed.

The number of cells migrated into the lower chamber under each condition can then be determined by a number of methods, including direct counting, labelling with fluorescent or radioactive markers or through the use of a vital dye. Typically, we utilise the vital dye MTT. 3 µl of stock MTT solution are added to each well, and then the plate is incubated at 37 °C for 1-2 hours during which time dehydrogenase enzymes within the cells convert the soluble MTT to an insoluble blue formazan product that can be quantified spectrophotometrically.

In parallel, an 8-point standard curve is set up. Starting with the number of cells added to each upper chamber (100,000) and going down in 2-fold serial dilutions in GBSS + BSA, the cells are added to a plate in 25 µl, with 3 µl of MTT stock solution added. The standard curve plate is incubated along side the migration plate.

At the end of this incubation, the liquid is carefully removed from the lower chambers, taking care not to disturb the precipitated formazan product. After allowing to air dry briefly, 20µl of DMSO is added to each lower chamber to solubilise the blue dye, and absorbance at 595nm is determined using a 96-well plate reader. The absorbance of each well is then interpolated to the standard curve to estimate the number of cells in each lower chamber.

The MCP-1 stimulated migration is determined by subtracting the average number of cells that reached the lower compartment in wells where no MCP-1 was added from the average number of cells that reached the lower compartment where MCP-1 was present at 25ng/ml.

The impact of the test substance is calculated by comparing the MCP-1-induced migration which occurred in the presence or absence of various concentrations of the test substance. Typically, the inhibition of migration is expressed as a percentage of the total MCP-1 induced migration which was blocked by the presence of the compound. For most compounds, a dose-response graph is constructed by determining the inhibition of MCP-1 induced migration which occurs at a range of different compound concentrations (typically ranging from 1nM to 1µM or higher in the case of poorly active compounds). The inhibitory activity of each compound is then expressed as the concentration of compound required to reduce the MCP-1-induced migration by 50% (the ED₅₀ concentration).

### Results

The compounds of examples 1 to 2 and reference examples 1 to 5 were tested and were shown to have an ED₅₀ of 100 nM or less in this test.

### Enantioselectivity

The (S)- and (R)- enantiomers of two different members of the aminocaprolactam series can be synthesised to determine whether the biological activity showed enantioselectivity.

The dose-response curves for each of the compounds as inhibitors of MCP-1 induced THP-1 cell migration can be determined using the transwell migration assay.

For the application of the compounds of the present invention as anti-inflammatory agents in vivo it is preferable to use the pure (S)-enantiomer of the compound, rather than the racemic mixture of the two enantiomers or the pure (R)-enantiomer.

## Claims

1. A compound of general formula (I) or a pharmaceutically acceptable salt thereof: 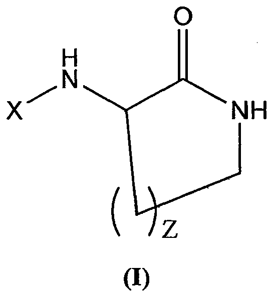 wherein
z is 1 or 2;
X is -CO-Yₖ-(R¹)ₙ;
k is 0 or 1;
Y is a cycloalkyl or polycyloalkyl group;
each R¹ is a branched chain alkyl group;
n is any integer from 1 to m, where m is the maximum number of substitutions permissible on the cyclo-group Y (such that n=1 if k=0, such that the R¹ group is bonded directly to the carbonyl group); and
the compound comprises an amino lactam ring linked to an alkyl group -Yₖ-(R¹)ₙ by an amide group, and wherein the carbon atom in the alkyl group at the 2-position relative to the carbon atom of the amide carbonyl is linked to each of the carbon atom of the amide carbonyl and three other carbon atoms by a single bond, and wherein the 2-position carbon atom has essentially tetrahedral bond angles.

2. The compound according to claim 1, wherein the compound is a compound of formula **(I')** or a pharmaceutically acceptable salt thereof: 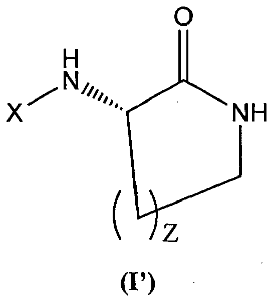 wherein X and Z are as defined in claim 1.

3. A pharmaceutical composition comprising, as active ingredient, a compound according to claim 1 of formula (I) or a pharmaceutically acceptable salt thereof, and at least one pharmaceutically acceptable excipient and/or carrier:

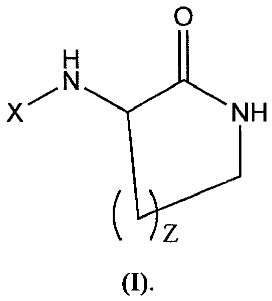

4. The pharmaceutical composition according to claim 3, wherein the compound is a compound according to claim 2 of formula (I') or a pharmaceutically acceptable salt thereof:

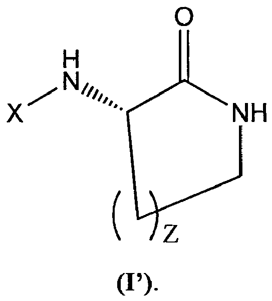

5. Use of a compound according to claim 1 of general formula (I): 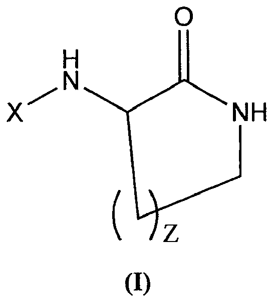 for the preparation of a medicament intended to treat an inflammatory disorder.

6. The use according to claim 5, wherein the compound is a compound according to claim 2 of general formula **(I'):** 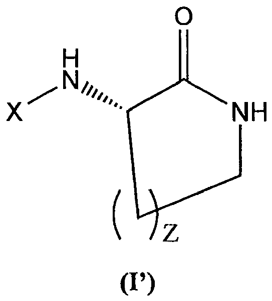 for the preparation of a medicament intended to treat an inflammatory disorder.

7. The compound according to claim 1 or 2 of general formula **(I)** or **(I'),** or a pharmaceutically acceptable salt thereof, wherein z=2.

8. The compound according to claim 2, wherein the compound is selected from the group consisting of:
- (*S*)-3-(2',2'-Dimethyldodecanoylamino)-tetrahydropyridin-2-one;
- (*S*)-3-(2',2'-Dimethyldodecanoylamino)-pyrrolidin-2-one;
and pharmaceutically acceptable salts thereof.

9. Use of a compound of formula **(I)** or **(I')** according to one of claims 5 and 6 wherein the inflammatory disorder is selected from the group consisting of autoimmune diseases, vascular disorders, viral infection or replication, asthma, osteoporosis (low bone mineral density), tumor growth, rheumatoid arthritis, organ transplant rejection and/or delayed graft or organ function, a disorder **characterised by** an elevated TNF-α level, psoriasis, skin wounds, disorders caused by intracellular parasites, allergies, Alzheimer's disease, antigen induced recall response, immune response suppression, multiple sclerosis, ALS, fibrosis, and formation of adhesions.

10. A compound according to claim 1 or 2 for use in the treatment of an inflammatory disorder.

11. The compound for use according to claim 10, wherein the inflammatory disorder is selected from the group defined in claim 9.

## Patentansprüche

1. Verbindung der allgemeinen Formel (I) oder ein pharmazeutisch akzeptables Salz davon: 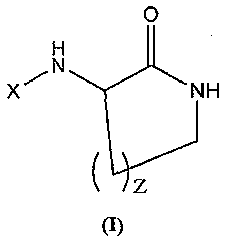 wobei
z 1 oder 2 ist;
X -CO-Yₖ-(R¹)ₙ ist;
k 0 oder 1 ist;
Y eine Cycloalkyl- oder Polycycloalkylgruppe ist;
jedes R¹ eine verzweigtkettige Alkylgruppe ist;
n eine beliebige ganze Zahl von 1 bis m ist, wobei m die maximale Anzahl von Substitutionen ist, die auf der Cyclo-Gruppe Y zulässig sind (so dass n=1 wenn k=0, so dass die R¹-Gruppe direkt an die Carbonylgruppe gebunden ist); und
die Verbindung einen Aminolactamring beinhaltet, der mit einer Alkylgruppe -Yₖ-(R¹)ₙ über eine Amidgruppe verknüpft ist, und wobei das Kohlenstoffatom in der Alkylgruppe an der 2-Position relativ zum Kohlenstoffatom des Amidcarbonyls mit jedem Kohlenstoffatom des Amidcarbonyls und drei anderen Kohlenstoffatomen über eine Einfachbindung verknüpft ist, und wobei das Kohlenstoffatom an der 2-Position im Wesentlichen tetraedrische Bindungswinkel hat.

2. Verbindung nach Anspruch 1, wobei die Verbindung eine Verbindung der Formel (I') oder ein pharmazeutisch akzeptables Salz davon ist: 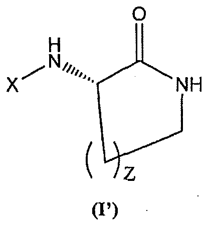 wobei X und Z wie in Anspruch 1 definiert sind.

3. Pharmazeutische Zusammensetzung, die als Wirkstoff eine Verbindung nach Anspruch 1 von Formel (I) oder ein pharmazeutisch akzeptables Salz davon und wenigstens einen pharmazeutisch akzeptablen Exzipienten und/oder Träger beinhaltet:

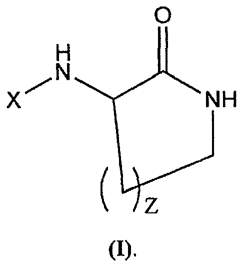

4. Pharmazeutische Zusammensetzung nach Anspruch 3, wobei die Verbindung eine Verbindung nach Anspruch 2 von Formel (I') oder ein pharmazeutisch akzeptables Salz davon ist:

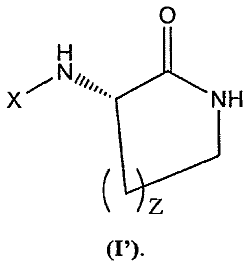

5. Verwendung einer Verbindung nach Anspruch 1 der allgemeinen Formel (I): 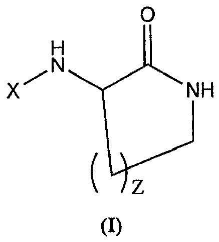 zur Herstellung eines Medikaments, das für die Behandlung einer Entzündungsstörung vorgesehen ist.

6. Verwendung nach Anspruch 5, wobei die Verbindung eine Verbindung nach Anspruch 2 der allgemeinen Formel (I') ist: 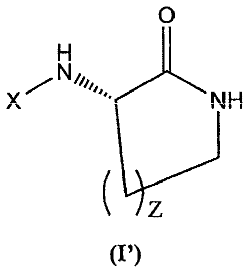 zur Herstellung eines Medikaments, das für die Behandlung einer Entzündungsstörung vorgesehen ist.

7. Verbindung nach Anspruch 1 oder 2 der allgemeinen Formel (I) oder (I') oder ein pharmazeutisch akzeptables Salz davon, wobei z=2 ist.

8. Verbindung nach Anspruch 2, wobei die Verbindung ausgewählt ist aus der Gruppe bestehend aus:
- (*S*)-3-(2',2'-Dimethyldodecanoylamino)-tetrahydropyridin-2-on;
- (*S*)-3-(2',2'-Dimethyldodecanoylamino)-pyrrolidin-2-on; und pharmazeutisch akzeptable Salze davon.

9. Verwendung einer Verbindung der Formel (I) oder (I') nach einem der Ansprüche 5 und 6, wobei die Entzündungsstörung ausgewählt ist aus der Gruppe bestehend aus Autoimmunkrankheiten, Gefäßstörungen, Virusinfektion oder -replikation, Asthma, Osteoporose (geringe Knochenmineraldichte), Tumorwachstum, Rheumatoidarthritis, Organtransplantatabstoßung und/oder verzögerte Transplantat- oder Organfunktion, einer Störung, die durch einen erhöhten TNF-α-Wert gekennzeichnet ist, Psoriasis, Hautwunden, Störungen infolge intrazellulärer Parasiten, Allergien, Alzheimer-Krankheit, antigeninduzierter Rückerinnerungsreaktion (Recall Response), Immunreaktionsunterdrückung, Multipler Sklerose, ALS, Fibrose und Entstehung von Adhäsionen.

10. Verbindung nach Anspruch 1 oder 2 zur Verwendung bei der Behandlung einer Entzündungsstörung.

11. Verbindung zur Verwendung nach Anspruch 10, wobei die Entzündungsstörung aus der in Anspruch 9 definierten Gruppe ausgewählt ist.

## Revendications

1. Composé de formule générale **(I)** ou sel pharmaceutiquement acceptable de celui-ci : 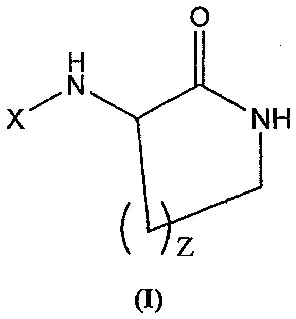 où
z est égal à 1 ou 2 ;
X représente -CO-Yₖ-(R¹)ₙ ;
k est égal à 0 ou 1 ;
Y représente un groupement cycloalkyle ou polycycloalkyle ;
chaque R¹ représente un groupement alkyle à chaîne ramifiée ;
n représente tout nombre entier compris entre 1 et m, où m est le nombre maximum de substitutions possibles sur le groupement cyclo Y (si bien que n = 1 si k = 0 et si bien que le groupement R¹ est directement lié au groupement carbonyle) ; et
le composé comprend un noyau aminolactame lié à un groupement alkyle -Yₖ-(R¹)ₙ par un groupement amide, et où l'atome de carbone du groupement alkyle en position 2 par rapport à chaque atome de carbone du carbonyle de l'amide est lié à l'atome de carbone du carbonyle de l'amide et à trois autres atomes de carbone par une simple liaison, et où l'atome de carbone en position 2 présente des angles de liaison tels que la géométrie est essentiellement tétraédrique.

2. Composé selon la revendication 1, où le composé est un composé de formule **(I')** ou un sel pharmaceutiquement acceptable de celui-ci : 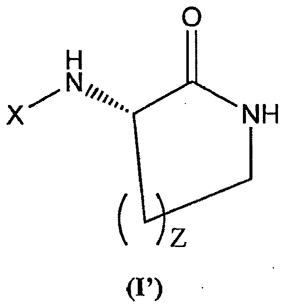 où X et Z sont tels que définis à la revendication 1.

3. Composition pharmaceutique comprenant, comme ingrédient actif, un composé de formule **(I)** selon la revendication 1 ou un sel pharmaceutiquement acceptable de celui-ci et au moins un excipient et/ou un véhicule pharmaceutiquement acceptable(s) :

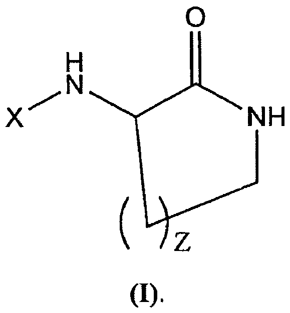

4. Composition pharmaceutique selon la revendication 3, où le composé est un composé de formule **(I')** selon la revendication 2 ou un sel pharmaceutiquement acceptable de celui-ci :

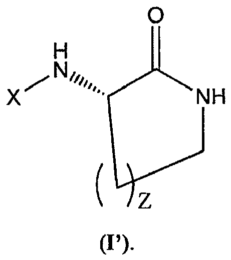

5. Utilisation d'un composé de formule générale **(I)** selon la revendication 1 : 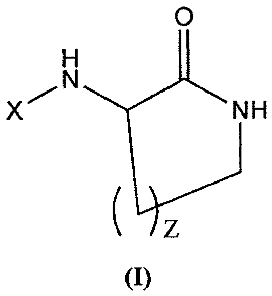 pour la préparation d'un médicament conçu pour traiter une affection inflammatoire.

6. Utilisation selon la revendication 5, où le composé est un composé de formule générale **(I')** selon la revendication 2 : 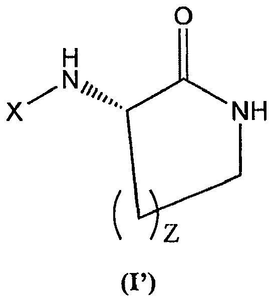 pour la préparation d'un médicament conçu pour traiter une affection inflammatoire.

7. Composé de formule générale **(I)** ou **(I')** selon la revendication 1 ou 2 ou sel pharmaceutiquement acceptable de celui-ci, où z = 2.

8. Composé selon la revendication 2, où le composé est sélectionné dans le groupe consistant en les suivants :
- (S)-3-(2',2'-diméthyldodécanoylamino)-tétrahydropyridine-2-one ;
- (S)-3-(2',2'-diméthyldodécanoylamino)-pyrrolidine-2-one ;
et sels pharmaceutiquement acceptables de ceux-ci.

9. Utilisation d'un composé de formule **(I)** ou **(I')** selon l'une des revendications 5 et 6, où l'affection inflammatoire est sélectionnée dans le groupe consistant en les suivantes : maladies auto-immunes, affections vasculaires, infection ou réplication virale, asthme, ostéoporose (faible densité minérale osseuse), croissance tumorale, polyarthrite rhumatoïde, rejet de greffe d'organe et/ou retard de fonction du greffon ou de l'organe, affection **caractérisée par** des taux élevés de TNF-α, psoriasis, plaies cutanées, affections causées par des parasites intracellulaires, allergies, maladie d'Alzheimer, réponse de rappel induite par un antigène, suppression de la réponse immunitaire, sclérose en plaques, SLA, fibrose et formation d'adhérences.

10. Composé selon la revendication 1 ou 2 pour une utilisation dans le traitement d'une affection inflammatoire.

11. Composé pour une utilisation selon la revendication 10, où l'affection inflammatoire est sélectionnée parmi le groupe défini à la revendication 9.


### SEQUENCE LISTING

<110> Cambridge university Technical services Limited Grainger, David J Fox, David J
<120> Anti-Inflammatory Agents
<130> JMD/CSW/48552.WO01
<150> GB0512238.7
   <151> 2005-06-15
<160> 1
<170> PatentIn version 3.2
<210> 1
   <211> 11
   <212> PRT
   <213> Artificial
<220>
   <223> Human protein analogue
<300>
   <301> Reckless et al.
   <302> Identification of oligopeptide sequences which inhibit migration induced by a wide range of chemokines
   <303> Biochemical Journal
   <304> 340
   <306> 803-811
   <307> 1999
<400> 1
   Cys Gln Ile Trp Lys Gln Lys Pro Asp Leu Cys
   1               5                   10